# EUROPEAN PATENT SPECIFICATION

(11) **EP 4 584 348 B1**
(45) Date of publication and mention of the grant of the patent: **01.04.2026**
(21) Application number: 23768269.5
(22) Date of filing: 07.09.2023
(51) Int. Cl.: C10G 9/36, C10G 9/00, F22B 1/00, F01K 3/18, F22B 1/18, F22G 1/02

(54) **METHOD AND SYSTEM FOR STEAM CRACKING**
VERFAHREN UND SYSTEM ZUM DAMPFKRACKEN
PROCÉDÉ ET SYSTÈME DE VAPOCRAQUAGE

(30) Priority: 09.09.2022 EP 22020434
(43) Date of publication of application: 16.07.2025
(73) Proprietor: Linde GmbH, 82049 Pullach (DE)
(72) Inventor: HELFENBEIN, Sebastian, 82049 Pullach (DE); ZELLHUBER, Mathieu, 82049 Pullach (DE); SINN, Tobias, 82049 Pullach (DE); HÖRENZ, Michael, 82049 Pullach (DE); FRITZ, Helmut, 82049 Pullach (DE)
(86) International application number: PCT/EP2023/074631
(87) International publication number: WO 2024/052486

(56) References cited:
- WO-A1-2021/052642
- US-A1- 2017 022 429
- US-A1- 2021 171 836

## Description

The present invention relates to a method and a system for steam cracking according to the preambles of the independent claims.

### Background of the invention

The present invention is based on the steam cracking technology for the production of olefins and other base chemicals, as e.g. described in the article "Ethylene" in Ullmann's Encyclopedia of Industrial Chemistry, online publication 15 April 2009, DOI: 10.1002/14356007.a10_045.pub2.

Presently, the thermal energy required for initiating and maintaining the endothermic cracking reactions in steam cracking is provided by the combustion of fuel gas in a refractory furnace. The process gas initially containing steam and the hydrocarbons to be cracked is passed through so-called cracking coils placed inside the refractory box, also called radiant zone or section. On this flow path the process gas is continuously heated, enabling the desired cracking reactions to take place inside the cracking coils, and thus the process gas is continuously enriched in the cracking products. Typical inlet temperatures for the process gas into the cracking coils are between 550 and 750°C, outlet temperatures are typically in the range between 800 and 900°C.

In addition to the radiant zone, fired cracking furnaces comprise a so-called convection zone or section and a so-called quench zone or section. The convection zone is usually positioned above the radiant zone and composed of various tube bundles traversing the flue gas duct from the radiant zone. Its main function is to recover as much energy as possible from the hot flue gas leaving the radiant zone. Indeed, only 35 to 50% of the total firing duty is typically transferred within the radiant zone to the process gas passed through the cracking coils. The convection zone therefore plays a central role in the energy management in steam cracking, as it is responsible for the beneficial usage of approximately 40 to 60% of the heat input into a furnace (i.e. of the firing duty). Indeed, when taking the radiant and convection zone together, modern steam cracking plants make use of 90 to 95% of the overall fired duty (based on the fuel's lower heating value or net calorific value). In the convection section, the flue gas is cooled down to temperature levels between 60 and 140°C before leaving the convection section and being released to the atmosphere via stack.

The flue gas heat recovered in the convection zone is typically used for process duties such as preheating of boiler feed water and/or hydrocarbon feeds, (partial) vaporization of liquid hydrocarbon feeds (with or without prior process or dilution steam injection), and superheating of process steam and high-pressure steam.

The quench zone is positioned downstream of the radiant zone along the main process gas route. It is composed of one or more heat exchanger units, having the main functions of quickly cooling the process gas below a maximum temperature level to stop the cracking reactions, to further cool down the process gas for downstream treatment, and to effectively recover sensible heat from the process gas for further energetic usage. In addition, further cooling or quenching can be realized via injection of liquids, e.g. by oil quench cooling when steam cracking liquid feeds.

The process gas heat recovered in the quench section is typically used for vaporizing high-pressure (HP) or super-high-pressure (SHP) boiler feed water (typical at a pressure range between 30 and 130 bar absolute pressure), and for preheating the same boiler feed water, before it being fed to a steam drum. Saturated high-pressure or super-high-pressure steam generated accordingly may be superheated in the convection zone (see above) to form superheated high-pressure or super-high-pressure steam, and from there may be distributed to the central steam system of the plant, providing heat and power for heat exchangers and steam turbines or other rotating equipment. The typical degree of steam superheating achieved in furnace convection zones lies between 150 and 250 K above the saturation temperature (dew point margin). Generally, steam cracking furnaces may operate with high-pressure steam (typically at 30 to 60 bar) or with super-high-pressure-steam (typically at 60 to 130 bar). For the sake of clarity in the description of the present invention, high-pressure-steam will be used for the entire pressure range between 30 and 130 bar, but also beyond this upper limit, since the present invention may include in embodiments usage of steam at pressures of up to 175 bar.

A method for quenching cracked gases and recovery of heat therefrom by cooling the gases in heat exchange with steam to increase a superheat level of the steam and subsequently recovering heat from the superheated steam is disclosed in US 4,107,226 A. According to US 4,617,109 A, combustion air for steam cracking furnaces is preheated by indirect heat exchange with medium pressure and low-pressure steam that has been expanded through steam turbines from high pressure steam produced in the hot section of an ethylene production plant.

Such air preheating is a well-known measure to reduce fuel usage in furnaces, and has come more into the focus in the context of increased efforts to reduce carbon dioxide emissions. Air preheating in steam cracking arrangements may have significant impact on heat balancing and recovery, due to reduced availability of flue gas heat and additional air preheating requirements.

It is an object of the present invention to further improve known steam cracking arrangements in terms of efficiency and resource utilization, particularly in scenarios in which components of such arrangements are partly or completely electrified, and in which a more advantageous air preheating may be performed.

### Disclosure of the invention

Against this background, the present invention proposes a method and a system for steam cracking with the features of the independent claims. Embodiments of the invention are the subject of the dependent claims and of the description that follows.

Before further describing the features and advantages of the present invention, some terms used in the description thereof will be further explained.

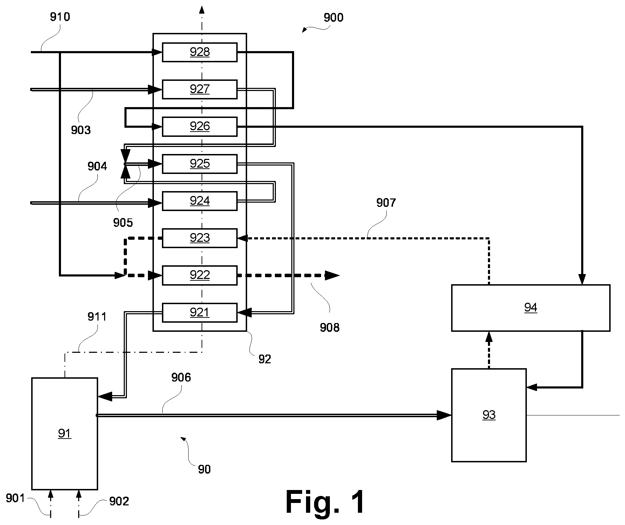

The terms "flue gas heat recovery section" and "convection section" may be used as synonyms herein. A flue gas heat recovery section or convection section may also include air preheating stages against flue gas, which are typically not included in the conventional convection zone design of furnaces without air preheating, as further illustrated in connection with Figure 1 below.

Likewise, the terms "cracked gas heat recovery section" and "quench section" may be used as synonyms herein. A cracked gas heat recovery section or quench section may include all multistream heat exchangers used to cool the cracked gas downstream of the cracking coil, but does not include direct contact cooling stages (i.e. cooling by liquid flow injections into the cracked gas stream in desuperheaters and/or quench columns) according to the understanding used here.

The terms "section(s)" and "zone(s)" and combinations thereof are used, in this connection, as synonyms herein as well.

A "multistream heat exchanger" is a heat exchanger configured to transfer heat between two or more media streams which are in indirect contact and separated by a heat exchange surface such as the wall of one or more chambers or tubes. Such multistream exchanger may e.g. be a quench exchanger as e.g. mentioned in the Ullmann article mentioned at the outset, or tube bundle exchanger placed in a flue gas channel, as typically done in a convection zone.

The term "feed" shall include liquid or gaseous hydrocarbons which may be evaporated, if necessary, and which are diluted or mixed with process or dilution steam, forming a "process gas" that is fed to the cracking furnace or radiant zone during a steam cracking operation. If reference is made to one of said streams being preheated, this may also include preheating a first partial stream and not preheating a second partial stream, particularly if said partial streams are later recombined to form a combined stream at a higher temperature as compared to the temperature before forming the partial streams.

The term "firing efficiency" is used as a synonym to the term "fuel-based radiant zone efficiency" and refers to the relative share or proportion of heat absorbed by the coils in the radiant section divided by the chemical energy converted into heat during fuel combustion in the radiant section, typically calculated in terms of lower heating value.

The prior art commonly discloses ways and methods to improve energy efficiency of cracking furnaces by foreseeing combustion air preheating. This is typically done by using internal heat from the flue gas system of the furnace using the preheated air, or using external heat from other sources (e.g. superheated or saturated steam, electricity, quench oil, etc.). Higher air preheating temperatures are generally targeted to further minimize the fuel gas consumption and therefore the carbon dioxide emissions of a furnace.

WO 2018/229267 A1 and WO 2021/052642 A1 propose a modified convection zone and quench section setup preferably used for fired furnaces with increased radiant zone efficiency (by air preheating or oxygen enrichment), in which the mixed hydrocarbon feed/process steam flow is at least directly preheated against cracked gas in a quench exchanger. According to said documents, individual embodiments feature only single-stage air-preheating systems, with air preheaters being arranged at the downstream end of the convection section, which limits the air preheating to moderate temperature levels. No air preheating temperatures are specifically mentioned, but these may be estimated, for technical reasons, to a maximum of 300 to 350°C.

Further straightforward options for air preheating in fired furnaces are external air preheating using external heating media, other then flue gas from combustion, like boiler feed water, steam, quench water, quench oil, thermo-oil, etc., or including a single air preheating stage at the downstream end of the convection section, which are not illustrated here for reasons of conciseness.

EP 0 229 939 B1 and US 3,426,733 A also disclose air preheating in steam cracking arrangements. Electrified steam cracking arrangements with electric or fired furnaces are also well known and are disclosed, for example, in WO 2020/150244 A1, WO 2020/150248 A1 and WO 2020/150249 A1.

US 2014/0212823 A1 mentions air preheat temperatures of up to ca. 482 °C (900 °F) that may be reached by internal air preheating against flue gas, but does not disclose comprehensive concepts for the heat recovery and energy balancing of such furnaces. It is indeed difficult to achieve a balanced energy integration for internal air preheating with temperatures above 400 °C when adopting conventional convection zone topologies like the ones shown in WO 2018/229267 A1 or WO 2021/052642 A1. The main reason is that in these topologies a large number of heat consumers are included, which cannot be sufficiently supplied with heat in case of such high air preheating temperatures. This is because for higher air preheating temperatures and higher radiant box efficiency, the available heat in the convection zone is reduced, even more if internal air preheating is foreseen.

Also, for external air preheating, a further increase of preheating temperatures is impeded due to the non-availability of suitable heating media at temperatures above 300 °C (the condensation temperature of super high pressure steam is at ca. 325 °C for a typical pressure of 120 barg (bar over atmospheric pressure)). Typical maximum air preheat temperatures found in the literature for steamcracking furnaces thus go up to around 300 °C when using external heat sources, see e.g. EP 0 229 939 B1. Also with electric air preheating it is conventionally not preferred to go beyond that range, as this would lead to very high electricity import quantities at a fairly low efficiency compared to a fully electrified furnace.

The present invention and embodiments thereof, in this connection, provide a fired cracking furnace heat recovery concept and corresponding process topologies specifically suited for higher air preheating temperatures, thus offering maximum firing efficiency/minimum fuel usage, while selectively recovering the waste heat for essential process purposes in the heat recovery sections of the furnace.

The present invention and embodiments thereof provide an advantageous solution to these problems, wherein a main idea or philosophy underlying embodiments of the present invention may be seen as maximizing a fired furnace efficiency by (i) prioritizing flue gas heat usage for maximum air preheating in the convection section, and (ii) simultaneously prioritizing cracked gas heat usage for maximum preheating of the process gas and/or the feed and/or steam used in forming the process gas in the quench section.

The approach of embodiments of the present invention may in principle pushing the furnace design into the direction of two separate feed-effluent cycles, namely (i) the combustion air/flue gas cycle and (ii) the process feed/cracked gas cycle. This means that an incremental lowering of fuel gas usage in a cracking furnace according to this invention is done by a simultaneous increase of the relative shares of flue gas heat usage for air preheating, and cracked gas heat usage for preheating of the process gas and/or the feed and/or steam used in forming the process gas.

The invention proposes, in this connection, a method of steam cracking using a steam cracking system comprising one or more fired radiant sections, wherein, during a steam cracking operation, a fuel is combusted with an oxidizer gas containing oxygen, such as combustion air, to heat the one or more fired radiant sections, forming a flue gas. An oxidizer gas may also be oxygen enriched air, or a gas or gas mixture rich in oxygen, such as "technical" oxygen. Only for reasons of conciseness, and without limitation, the subsequent explanations focus on combustion air.

A process gas is formed from a (gaseous or liquid) hydrocarbon feed and (dilution or process) steam and said process gas is passed through one or more cracking coils in the one or more fired radiant sections, forming a cracked gas, wherein at least a part of the flue gas is cooled in a flue gas heat recovery section, wherein at least a part of the cracked gas is cooled in a cracked gas heat recovery section, wherein the flue gas heat recovery section and the cracked gas heat recovery section each comprise one or more heat exchangers, wherein the flue gas heat recovery section comprises heat exchange structures which are provided in an amount and a configuration such as to use, at least during a part of the steam cracking operation, a first proportion of more than 35% of a total amount of heat recovered within the one or more heat exchangers of the flue gas heat recovery section from the flue gas for preheating the oxidizer gas, and wherein the cracked gas heat recovery section is configured such as to use, at least during the same part of the steam cracking operation, a second proportion of more than 35% of a total amount of heat recovered within the one or more heat exchangers of the cracked gas heat recovery section for preheating the process gas and/or the hydrocarbon feed and/or the steam used in forming the same.

The term "heat exchange structures" may include any technical means configured to enable or promote a heat transfer between different media, such as, for example, straight or wound coils, and a "configuration" of the heat exchange structures may particularly include a heat exchange surface and/or technical modifications such as, for example, fins on the outer surface of a heat exchanger coil and/or grooved or finned channels on the inner surface of a heat exchanger coil and/or any other surface-increasing feature on the inner or outer surface of a heat exchanger coil.

The heat exchangers in the flue gas heat recovery section and the cracked gas heat recovery section mentioned are particularly multistream heat exchangers such as mentioned before, and particularly at least a major proportion of a total amount of heat transferred in the flue gas heat recovery section and the cracked gas heat recovery section is transferred in such multistream heat exchangers. That is, the proportions mentioned before do generally not relate to, or include, heat recovered otherwise such as by injection of media or direct contact cooling, even if such injection or direct contact cooling may also be performed in embodiments of the present invention in the flue gas heat recovery section and the cracked gas heat recovery section. If the latter is the case, however, generally only a small amount of fluids, as compared to the flue gas or cracked gas, is injected into the flue gas heat recovery section and the cracked gas heat recovery section, such as an amount of less than 10%, 5%, 0,5%, 1% or 0,1% as compared to the flue gas or cracked gas.

In each case, preheating the oxidizer gas may include passing the whole oxidizer gas stream through a heat exchanger, but may also include heating a partial stream of the oxidizer gas and reuniting such a partial stream with a remainder, to form a combined stream at a temperature resulting from the mixing. Heating in the flue gas heat recovery section and the cracked gas heat recovery section may be performed serially or in parallel.

Advantages of the present invention include that, according to the present invention, realisation of high air preheating levels and therefore high radiant zone efficiencies for both gas and liquid feedstock crackers becomes possible. The result is a generally lower fuel gas usage, which is particularly beneficial in terms of carbon dioxide emissions, as mentioned for "scope 1" to "scope 3" emissions below.

This results in a reduced energy demand for the cracking furnace or system itself. This advantage is independent of the applied fuelling technique, such that the present invention can, in embodiments, be combined with any currently available or upcoming combustion technology. High temperature combustion air or alternative oxidizer gas heated with flue gas is a furnace-internal energy carrier itself, which recycles energy from the convection section to the firebox of the cracking furnace. Thus, the presented invention optimises any furnace combustion system, independent of the fuel/oxidizer mixture sent to the burners. This also includes oxygen-enriched air, hydrogen enriched or pure hydrogen fuel, ammonia fuel and others. According to the present invention, a heat exchange between thermodynamically "similar" material flows with similar specific heat capacity curves over temperature and similar flow rates (flue gas to combustion air and cracked gas to process gas, i.e. feed and/or steam) may be optimized. Embodiments of the present invention are highly compatible with highly electrified separation train designs demanding significantly less steam with reduced superheating due to absence of large steam turbines. An inherent safety is provided because of a heat exchange between "compatible" media without explosion/deflagration risk in case of tube rupture (by avoiding e.g. air preheat vs. cracked gas, but also by minimizing/avoiding process gas or feed preheat vs. flue gas).

The solutions proposed according to the present invention significantly deviate from the prior art such as represented by WO 2018/229267 A1 and WO2021052642A1, as these documents propose process setups which are mainly aimed at increasing only the share of cracked gas waste heat usage for process gas preheating. Indeed, these documents disclose embodiments with and without air preheating, with process sequences for the process gas preheating arranged independently of the provision of air preheating in each of the two documents. The solutions proposed in both documents WO 2018/229267 A1 and WO 2021/052642 A1 are built on the core feature of executing the initial cracked gas cooling in a feed-effluent exchanger against a process gas stream, which was previously preheated in the convection section and which may be preheated further in the convection zone of the furnace. It is clear that the prior art represented by these documents is aimed at the combination of their proposed process gas preheating sequences with only low levels of air preheating. This is clearly stated e.g. in these documents. The prior art therefore proposes no strict correlation between waste heat usages in convection and quench sections.

According to embodiments of the present invention, the first proportion and the second proportion may be, independently from each other, proportions of more than 35%, more than 40%, more than 45%, or more than 50%, such that, in such embodiments, the advantages of the present invention may be selected to be more or less pronounced, by using either lower or higher proportions.

The oxidizer gas may, in embodiments of the present invention, be preheated to a temperature exceeding 400 °C, 500 °C, 600 °C, or 700°C, which are temperature levels significantly exceeding those known from the state of the art.

The oxidizer gas, or a part thereof, is at least partially preheated in a minimum of two separate oxidizer gas preheating stages against the flue gas in the flue gas heat recovery section, and the flue gas may be used in between the two oxidizer gas preheating stages to superheat high pressure steam at a pressure level between 30 and 175 bar absolute pressure.

Placing the second oxidizer gas preheating stage upstream of a high pressure steam superheating bundle in the convection section leads to a significant change in convection section heat balance, and therefore the oxidizer gas preheating is prioritized over export steam generation and is consequently placed in the upstream part of the flue gas pathway.

Steam may, in embodiments of the present invention, be superheated in the flue gas heat recovery section, wherein said superheating of steam in the flue gas heat recovery section is limited to a maximum temperature of 450 °C, 400 °C. or 350°C and/or resulting in a dew point margin of maximally 150 K, 120 K, 80 K, or 60 K, wherein no further steam superheating is performed before exporting the steam from the flue gas heat recovery section or the steam cracking system, and wherein less than 25 %, 23 %, or 21 % of the heat recovered in the flue gas heat recovery section is used for generating high-pressure export steam. By reducing the steam superheating level and/or maximum temperature, in such an embodiment, a steam superheating can be more easily placed downstream of a high-temperature oxidizer gas preheating section in the flue gas pathway.

Steam exported from the flue gas heat recovery section or the steam cracking system is, in embodiments of the present invention, passed in a proportion of more than 60%, 70%, or 80% via conduits, pressure-reducing valves and/or nozzles, with or without prior desuperheating, to one or more steam-condensating heat exchangers. In particular, for this major part of the exported steam flow, any type of pressure reduction upstream of said heat exchangers is done without extraction of usable mechanical work, unlike it would be the case, e.g., in a turbine. Simultaneously, for a remaining minor part of the exported steam flow, after an optional first pressure reduction, e.g. from a very high pressure level via a valve to a high pressure level thereby yielding a steam flow at lower pressure but higher superheating level, a further pressure reduction may be realized e.g. by using backpressure turbines and extracting mechanical work from the steam flow. The resulting, expanded steam flow is then preferably routed to steam-condensating heat exchangers operated at a corresponding lower pressure level. The export of only moderately superheated steam from the furnaces is aimed at primarily providing energy to heat consumers, without significant extraction of mechanical work from the export steam. The overall steam quantity and specific enthalpy is hence reduced in such a setup.

In embodiments of the present invention, steam exported from the flue gas heat recovery section or the steam cracking system is not used in steam turbine drives delivering shaft powers of more than 5, 3, or 1 Megawatts. Consequently, the large rotating machinery in the downstream separation section is preferably driven by electric motors, "electrifying" such a setup and therefore reducing steam demand and (local) emissions from fired steam generation devices.

The process gas and/or the hydrocarbon feed and/or process steam used in forming the same may be heated using recovered cracked gas at least partially using a feed-effluent exchanger, and the feed-effluent exchanger may, in embodiments of the present invention, particularly be located downstream of a steam-generating quench exchanger in a flow path of the cracked gas. Generally, heat recovery from the cracked gas for preheating the process gas and/or the hydrocarbon feed and/or process steam used in forming the same may be performed using different topologies and process sequences. In most embodiments shown herein, the initial cracked gas cooling is done via a conventional steam-generating primary quench exchanger. Alternatively, an embodiment with a feed-effluent primary quench exchanger may also be provided according to embodiments of the present invention.

Saturated steam generated in a quench exchanger is used to preheat the process gas and/or the steam and/or the hydrocarbon feed used in forming the same, and a steam condensate collected from such preheating may be used for additional preheating of the same media at a lower temperature level. The indirect usage of cracked gas heat for process gas, steam and/or feed preheat via intermediate steam generation is a well suited design option to guarantee fast and reliable cracked gas cooling in state-of-the-art steam generators, thereby circumventing potential challenges in the design and operation of direct feed-effluent exchanger design. According to the present invention, a flexible use of indirect cracked gas heat recovery for feed preheating becomes possible, which is particularly advantageous e.g. in the context of flexible feed steam cracking systems.

Using saturated steam as well as condensates from this steam further limits the requirement to superheat export steam in the convection section, and therefore facilitates the achievement of high oxidizer gas preheating duties in the flue gas section. Furthermore, such indirect heat recovery is well suited for revamps of existing plants, where an increased usage of cracked gas heat for process gas, steam and/or feed preheating may be achieved without significant modification of the existing quench coolers and steam drum.

In an embodiment of the present invention, no preheating of the process gas and/or the process steam and/or the hydrocarbon feed used in forming the process gas is performed within flue gas heat recovery section. The hot flue gas exiting the radiant section is therefore directly used for oxidizer gas preheating without prior heat transfer to a feed preheating bundle in the convection zone, in such an embodiment.

A final preheating of a mixture of the steam and the hydrocarbon feed, i.e. the process gas, or a part thereof, may in an embodiment be performed using an electrical superheater, before the mixture enters one or more cracking coils in one or more fired radiant sections.

The fuel gas usage from a system according to embodiments of the present invention may be reduced by a minimum of 30 % or 35 % or 40% or 45%, compared to conventional fired furnaces, the latter being operated without oxidizer gas preheating and using a conventional fuel mixture of tail gas and natural gas import.

In embodiments of the present invention, a control system may be provided which is adapted to control properties of one or more further streams. For example, using such a control system, a flue gas cooling below condensation temperature and consequent corrosion issues may be avoided. Equipmentwise, a backup oxidizer gas preheater can be installed upstream of the oxidizer gas preheater(s) in the convection section and only be operated in special scenarios with any sort of waste or low temperature heat from the process plant. For example, a bypass controller on oxidizer gas or flue gas side may be provided, or any additional equipment.

In embodiments of the present invention, saturated steam generated in a quench exchanger may be further used to preheat fuel gas, and the steam condensate collected from this or other preheating steps may be used for additional preheating of fuel gas at a lower temperature level. This usage of saturated steam as heat provider may also be used for any other heating application for process streams, but in particular in vicinity of the furnace section to avoid long transfer lines carrying a saturated steam flow (risk of liquid deposits in piping and/or steam hammering).

In embodiments of the present invention, no heat from the flue gas is used to generate steam in a "boiler" bundle. Furthermore, when air is used as oxidizer gas, the same may be further enriched with by adding an oxidizer stream with superatmospheric oxygen content from an external source, e.g. electrolyzer, cryogenic air separation unit, or (vacuum) pressure swing adsorption unit.

The steam cracking system can, in embodiments of the present invention, be operated in energy-flexible manner, i.e. a given chemical production load of a furnace can be provided at differing total fuel gas consumption rates, e.g. by varying the air preheating temperature through use of air bypass lines around one or both of the air preheating stages. In embodiments of the present invention, no boiler feed water preheating is done against flue gas from the furnace The fuel gas consumed in the radiant zone may contain fossil-based and/or synthetic and/or bio-based hydrocarbons, fossil-based (grey/blue/turquoise) and /or renewable-based (green) hydrogen and/or ammonia. The composition of the supplied fuel gas may be varied during operation of the furnace along with other critical furnace operation parameters. Particularly, an increase in content of carbon-neutral fuels (e.g. hydrogen, ammonia) may be done in combination with taking measures to maximize the air preheat temperature, in order to minimize the consumption of such high-value clean fuels. The adjustment of fuel composition may be done by variation of a hydrogen separation in the tail gas processing and/or by variation of external fuel import quantities.

A steam cracking system comprising one or more fired radiant sections and means configured to combust, during a steam cracking operation, a fuel with an oxidizer gas to heat the one or more fired radiant sections, forming a flue gas, means configured to to form a process gas from a hydrocarbon feed and process steam, means configured to pass the process gas through one or more cracking coils in one or more fired radiant sections, forming a cracked gas, a flue gas heat recovery section configured to cool at least a part of the flue gas, and a cracked gas heat recovery section configured to cool at least a part of the cracked gas is also provided. The flue gas heat recovery section and the cracked gas heat recovery section each comprise one or more multistream heat exchangers, wherein the flue gas heat recovery section comprises heat exchange structures which are provided in an amount and a configuration such as to use, at least during a part of the steam cracking operation, a first proportion of more than 35% of a total amount of heat recovered within the one or more multistream heat exchangers of the flue gas heat recovery section from the flue gas for preheating the oxidizer gas, and wherein the cracked gas heat recovery section is configured such as to use, at least during the same part of the steam cracking operation, a second proportion of more than 35% of a total amount of heat recovered within the one or more multistream heat exchangers of the cracked gas heat recovery section for preheating the process gas and/or the hydrocarbon feed and/or the steam used in forming the process gas.

For further details in relation to the steam cracking system provided according to the present invention and preferred embodiments thereof, reference is made to the explanations relating to the inventive method and its preferred embodiments above. Advantageously, the proposed arrangement is adapted to perform a method in at least one of the embodiments explained before in more detail.

To summarize and partly reiterate what was said above, the present invention particularly allows for a realisation of high air preheating levels and therefore high radiant zone efficiencies for both gas and liquid feedstock crackers. This results in low fuel gas usage, which is particularly beneficial in terms of carbon dioxide emissions: So-called "scope 1" emissions are achieved e.g. by reducing combustion of carbonaceous fuel, and/or reduction of "scope 2" emissions are achieved e.g. by using less hydrogen from an on-site electrolyser with consequently lower electric power consumption, and/or reduction of "scope 3" emissions are achieved e.g. by increased export of unused hydrogen fuel gas.

This results in a reduced energy demand for the cracking furnace itself. This advantage is independent of the applied fuelling technique, such that the presented invention can be combined with any currently available or upcoming combustion technology.

The present invention allows for the use of gaseous hydrocarbon feeds such as e.g. ethane, propane or butane, as well as for the use of liquid hydrocarbon feeds such as e.g. naphtha, gasoil or plastic waste pyrolysis oil. Embodiments of the present invention may be used for processing mostly one preferred hydrocarbon feed type in a given furnace, e.g. ethane or naphtha. Other embodiments of the present invention may also use (variable) mixtures of gaseous hydrocarbon feeds, e.g. ethane with propane, or (variable) mixtures of liquid hydrocarbon feeds, e.g. naphtha with plastic waste pyrolysis oil. In further embodiments, cracking operation may include differing operation periods with alternating use of liquid and gaseous hydrocarbon feeds in a single feed-flexible furnace. Moreover, the present invention may also be applied in furnaces, in which at least temporarily a simultaneous co-cracking of gaseous and liquid hydrocarbon feed streams is performed.

The present invention may further be combined with any type of additional flue gas treatment step that is used to (further) reduce the concentration of environmentally relevant components in the flue gas, e.g. to reduce the carbon dioxide content in a carbon capture process unit using an amine wash or equivalent treatment technology, and/or to reduce nitrous oxide emissions in a nitrous oxide removal unit using selective catalytic reduction or equivalent treatment technology.The implementation of such additional flue gas treatment steps is facilitated by the invention through the reduced fuel gas usage and consequently reduced flue gas quantities.

According to embodiments of the present invention, a high temperature of combustion air or alternative oxidizer gas heated with flue gas, which is a furnace-internal energy carrier itself, is achieved, which recycles energy from the convection section to the firebox of the cracking furnace. Thus, the presented invention optimises any furnace combustion system, independent of the fuel/oxidizer mixture sent to the burners. This also includes oxygen-enriched air or oxidizer gas made of pure oxygen with or without dilution by recirculated flue gas, hydrogen enriched or pure hydrogen fuel, ammonia fuel and others, according to embodiments of the invention.

Embodiments of the present invention result in an optimized heat exchange between thermodynamically "similar" material flows with similar specific heat capacity curves over temperature and similar flow rates (flue gas to combustion air and cracked gas to feed). A high compatibility with a highly electrified separation train design demanding significantly less steam with reduced superheating due to absence of large steam turbines may furthermore be achieved in embodiments of the invention.

Embodiments of the present invention are inherently safe because of heat exchange between "compatible" media without explosion/deflagration risk in case of tube rupture by avoiding e.g. air preheat vs. cracked gas, but also by minimizing/avoiding process gas or feed preheat vs. flue gas. A flexible use of indirect cracked gas heat recovery for feed, process gas or process steam preheating is advantageous e.g. in the context of flexible feed crackers.

Embodiments of the present invention may also include the use of electric heaters at other locations than for the final preheating stage of the process gas. Electric heaters may in particular be used for process steam superheating before mixing with the hydrocarbon feed stream, and/or oxidizer gas preheating, and/or superheating of high-pressure export steam. Such electric heaters may also be configured for temporary or flexible usage, to compensate for fluctuating preheating duty demands linked e.g. to fouling/coking of multistream heat exchangers and/or variable feed supply conditions. Electric oxidizer gas preheaters may also be operated preferably in situations with renewable electricity surplus in the grid, to temporarily reduce fuel gas demand and increase fuel gas storage levels in the plant.

The present invention can further be combined with dedicated energy storage systems, e.g. latent heat storage systems or similar. In particular, at least a part of the exported high-pressure steam may temporarily be routed to suitable heat storage systems.

Steamcracker plants, featuring fired furnace systems according to the present invention, may further be integrated into a hybrid system architecture, in which fired furnaces according to the present invention are operated alongside with electrically heated furnaces (independently of the electric coil heating principle: direct resistive coil heating, indirect radiative coil heating by electric heating elements, coil heating using inductive power transmission). The steamcracker plant might further include other units for steam generation from electric energy (e.g. electric heat pump systems, electric boilers, etc.)

The invention is preferably combined with a separation train, in which at least a large proportion of more than 50%, 70%, 90% or 95% of the molecular hydrogen, contained in the cracked gas and possible additional feed streams to the separation section of the plant, is separated as a purified stream with a molar hydrogen content of more than 95 % and used for firing in the furnaces.

All features proposed herein for embodiments of the present invention may individually or in any grouping be combined with features proposed in International Patent Application PCT/EP2022/055877 and European Patent Application 21161768.3,

The present invention and embodiments thereof are further explained in connection with the appended drawings.

### Short description of the Figures

Embodiments of the invention will now be described, by way of example only, with reference to accompanying drawings, in which
Figure 1 illustrates an embodiment not forming part of the present invention,
Figure 2 illustrates basic principles of embodiments of the present invention, and
Figures 3 to 8 illustrate embodiments of the present invention.

### Embodiments

In the Figures, elements of identical, essentially identical, functionally comparable, or technically compatible function and/or purpose may be identified with identical reference numerals, and repeated explanations may be omitted for reasons of conciseness. Explanations herein relating to devices, apparatus, arrangements, systems, etc., according to embodiments of the present invention likewise may apply to methods, processes, procedures, etc. according to embodiments of the present invention and vice versa.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims or limitations on equivalents to the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein. In addition, this disclosure may include other inventions not presently claimed, but which may be claimed in future, particularly when encompassed by the scope of the independent claims.

In the Figures, elements of identical, essentially identical, functionally comparable, or technically compatible function and/or purpose may be identified with identical or commonly incremented reference numerals, and repeated explanations may be omitted for reasons of conciseness. Explanations herein relating to devices, apparatus, arrangements, systems, etc., according to embodiments of the present invention likewise may apply to methods, processes, procedures, etc. according to embodiments of the present invention and vice versa.

When reference is made to "combustion air" hereinbelow, this is intended to encompass further oxidizer gases as well, such as mentioned above.

Further not shown in the Figures are one or more air compressing devices typically located upstream of the combustion air preheating section, which compensates for the pressure drop of the combustion air preheat exchangers. The preferred pressure on the firing side of the radiant section lies in the typical slightly subatmospheric range as in conventional cracking furnaces without combustion air preheating. Consequently, a flue gas blower/compression device would most preferably be located downstream of the convection section outlet.

For reference, and to further illustrate the background of the invention, a non-inventive fired steam cracking arrangement is illustrated in Figure 1 in a highly simplified, schematic partial representation and is designated 900.

The steam cracking system 900 illustrated in Figure 1 comprises one or more cracking furnaces 90. For conciseness only, "one" cracking furnace 90 is referred to in the following, while typical steam cracking arrangements 900 may comprise a plurality of cracking furnaces 90 which can be operated under the same or different conditions. Furthermore, each of the cracking furnaces 90 may comprise one or more of the components explained below.

The cracking furnace 90 comprises a radiant zone 91 and a convection zone 92. In embodiments different from the one shown in Figure 1, several radiant zones 91 may be associated with a single convection zone 92, etc.

In the example illustrated, several heat exchangers 921 to 928 are arranged in the convection zone 92, either in the numbers, arrangement or sequence shown, or in different numbers, arrangement or sequence. These heat exchangers 921 to 928 are typically provided in the form of tube bundles passing through the convection zone 92 and are positioned in the flue gas channel 911 from the radiant zone 91 which is illustrated with a dash-dotted arrow.

In the example illustrated, the radiant zone 91 is heated by means of a plurality of burners, which are not illustrated for reasons of conciseness, and which may be arranged on the floor and/or wall sides of a refractory forming the radiant zone 91. As illustrated with two dash-dotted arrows, a fuel gas 901 and an oxygen containing oxidizer gas 902, such as air, oxygen, enriched air etc., may be provided to the burners in the radiant zone 91.

In the example illustrated, a gaseous or liquid hydrocarbon stream 903, which is illustrated with a double arrow, is provided to the steam cracking system 900. It is also possible to use several hydrocarbon streams in the manner shown or in a different manner. The hydrocarbon stream is initially passed to the heat exchanger 927 in the convection zone 92 and preheated therein. Heat exchanger 927 is also referred to as a feed preheater. Process steam 904, which is likewise illustrated with a double arrow, is provided and, in the example illustrated, passed through the convection zone 92 or, more precisely, the heat exchanger 924, where it is superheated. Heat exchanger 924 is therefore also referred to as a process steam superheater. Such process steam superheaters are typically used in cracking furnaces operated, at least partially, with liquid hydrocarbon feed streams, to facilitate the feed evaporation by adding strongly superheated process stream. Cracking furnaces operated solely with gaseaous hydrocarbon feed streams do typically not include such a process steam superheater.

The preheated hydrocarbon stream and the superheated process steam are combined to form a process gas stream 905 which is, as still illustrated with a double arrow, passed through heat exchanger 925 and thereafter through heat exchanger 921, these heat exchangers 925 and 921 also being referred to as first high temperature coil (heat exchanger 925) and second high temperature coil (heat exchanger 921), and subsequently through the one or more cracking coils in the radiation zone 91.

A product mixture 906 obtained in the radiant zone 91, which is also referred to as cracked or raw gas, is passed out of the radiation zone 91 and through a primary quench exchanger 93, which is shown here as a super high-pressure steam generating arrangement , before being passed on to subsequent treating and separation steps generally known from the art which are also not illustrated.

The steam generating arrangement 93 is associated with a super high-pressure steam drum 94. Boiler feed water 910 may, as illustrated with solid arrows throughout, be preheated in the heat exchangers 928 and 926 in the convection section 92, which are also referred to as first economizer (heat exchanger 928) and second economizer (heat exchanger 926), and thereafter fed into the super high-pressure steam drum 94 and the super high-pressure steam generating arrangement 93.

From the super high pressure steam generating arrangement 93, saturated super high pressure steam 907, as illustrated with finely dotted arrows, is withdrawn, passed through super high pressure steam drum 94, and superheated in heat exchangers 923 and 922 in the convection zone 92, these heat exchangers 923 and 922 also being referred to as first super high pressure steam superheater (heat exchanger 923) and second super high pressure steam superheater (heat exchanger 922), forming superheated super high pressure steam 908, as illustrated with coarsely dotted arrows. At a position between heat exchangers 923 and 922, an injection of boiler feed water 910 may also be performed.

In a non-inventive setup of a steam cracking system 900 as illustrated in Figure 1, the heat recovered from cracked gas cooling, such as in a primary quench exchanger 93 or elsewhere, is typically exclusively used to generate very high pressure export steam, while the heat recovered in the flue gas section, i.e. in the convection zone 92, is used to preheat the feed as illustrated, as well as to generate very high pressure export steam (by preheating boiler feed water and superheating saturated super high pressure steam).

The design philosophy underlying embodiments of the present invention includes, as mentioned before and as will be explained for various embodiments below, maximizing the efficiency of a fired furnace by (i) prioritizing flue gas heat usage for maximum air preheating in the convection section, and (ii) simultaneously prioritizing cracked gas heat usage for maximum process gas, feed and/or process steam preheating in the quench section

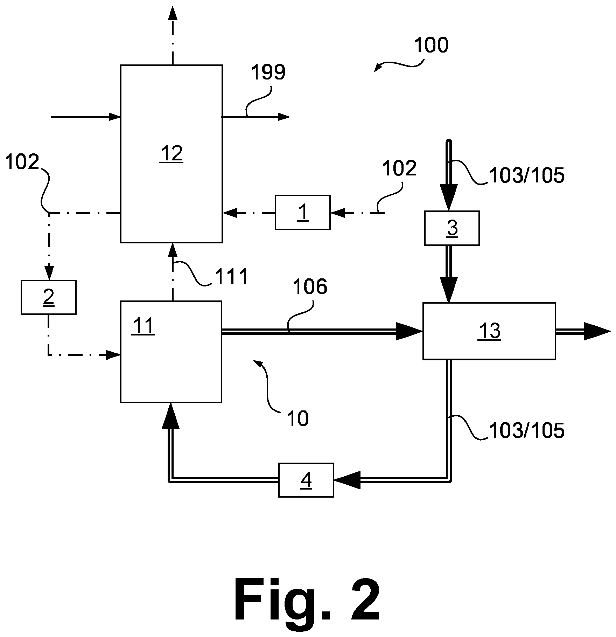

In Figure 2, basic principles of embodiments of the present invention are schematically illustrated in a strictly simplified view of a steam cracking system 100.

A cracking furnace 10 illustrated in Figure 2 comprises a radiant zone 11 and a convection zone 12. In other embodiments than the one shown in Figure 1, also several radiant zones 11 may be associated with a single convection zone 12, etc., as explained above for the system 900 in connection with Figure 1. A cracked gas heat recovery section is shown as 13.

In the example illustrated, several heat exchangers may be arranged in the convection zone 12, but are not shown in Figure 2 for reasons of generality.

Like heat exchangers 921 to 928 in system 900 shown in Figure 1, these heat exchangers are typically provided in the form of tube bundles passing through the convection zone 12 and are positioned in the flue gas stream from the radiant zone 11. Alternative designs of multistream heat exchangers may also be used, in particular for the heat exchange between flue gas and an oxidizer gas. The convection zone 12 may comprise one or more straight flue gas channels as well as, if applicable, connecting ducts between multiple straight flue gas channels or connecting a straight flue gas channel via e.g. a flange connection to a multistream heat exchanger with a distinct outer shell. In the sense of the present invention, such a separately arranged multistream heat exchanger recovering heat from the flue gas stream forms also part of the flue gas heat recovery section.

Like explained for system 900 shown in Figure 1, the radiant zone 11 is heated by means of a plurality of burners, which are not illustrated for reasons of clarity, and which may be arranged on the floor and/or wall sides of a refractory forming the radiant zone 11. A fuel gas, which is not illustrated, and an oxygen containing oxidizer gas 102, such as air, which is indicated by dash-dotted arrows, may be provided to the burners in the radiant zone 11. A flue gas 111, as illustrated with a dash-dotted arrow as well, is passed through convection zone 12 which therefore is also referred to as a flue gas heat recovery unit. The oxygen containing oxidizer gas 102 is heated in the flue gas heat recovery unit 12 and in optional further heating steps 1 and 2 which may be realized according to embodiments of the present invention.

In the example illustrated, a gaseous or liquid hydrocarbon feed 103, or a mixture 105 of such a feed with process steam, i.e. a process gas in the language used herein, which is illustrated with a double arrow, is provided to the steam cracking system 100 as well. It is also possible to use several hydrocarbon streams, as explained for system 900 shown in Figure 1.

Such a hydrocarbon feed 103 or a process gas 105 is preheated, in the example shown, at least in a cracked gas heat recovery section 13, and optionally in further heating steps 3 and 4 therebefore and thereafter, and passed through the radiant section 11, forming a cracked gas 106 which is likewise illustrated as a double arrow. In certain embodiments of the present invention, the further heating steps 3 and 4 may also be done in a multistream heat exchanger which is part of the convection zone 12.

The cracked gas 106 is cooled in cracked gas heat recovery section 13 thereafter. One or more heat consumers other than air or oxygen containing oxidizer gas may be passed through convection section 12 as well, as illustrated with 199, and one or more heat consumers other than hydrocarbon feed may be passed through cracked gas heat recovery section 13, as not specifically indicated.

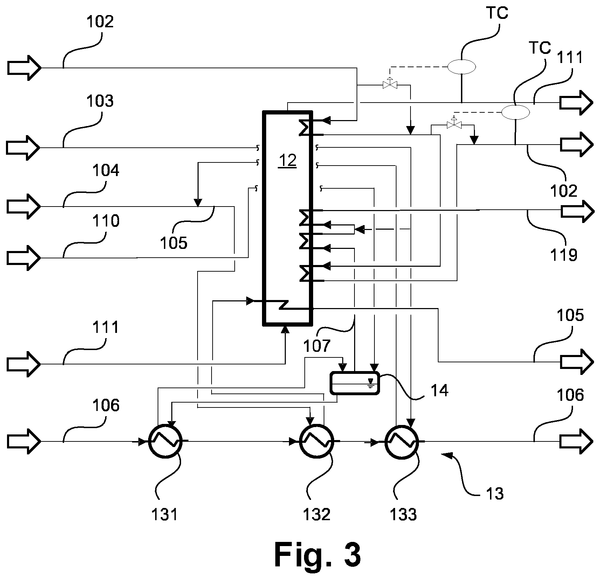

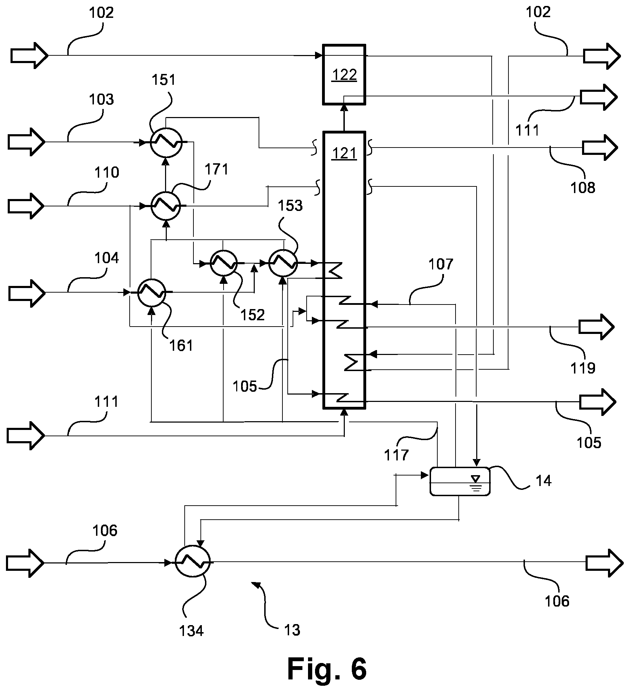

Figures 3 to 8 show embodiments of the present invention, for both gas feed steam cracking systems (Figures 3, 4 and 5, also referred to as embodiments 1, 2 and 3) and liquid feed steam cracking systems (Figures 6, 7 and 8, also referred to as embodiments 4, 5 and 6). Additional process data is provided in Tables 1 and 2, wherein comparative data to prior art embodiments with and without conventional, "low temperature" air preheating (APH) are also given. In the tables, "feed preheating" may refer to process gas and/or hydrocarbon feed and/or process steam preheating and as to the concepts realized, reference is made to Figures 3 to 8.

As shown in Figures 3 to 8, in preferred embodiments, the combustion air preheating is at least partially done in a minimum of two separate air preheating stages against flue gas. In between two air preheating stages, heat is preferably recovered from the flue gas to superheat steam, preferably generated in a steam generating quench exchanger. In some embodiments, in between two air preheating stages, heat may also additionally be recovered from the flue gas to heat other streams, e.g. to preheat process gas streams, as shown in Figure 6 for an embodiment comprising a liquid feed cracker setup.

Embodiments of the present invention may be applied in analogous manner for furnace systems operating with gaseous or liquid feedstock, by using slightly differing quench cooling and convection section sequences, while maintaining and making specific use of features of the present invention. This is specifically worth mentioning, given the differentiating process constraints in gas and liquid feed furnaces: The preheating duty in liquid feed furnaces is much larger than in gas feed furnaces, due to the additional vaporization enthalpy of the hydrocarbon feed. Moreover, less heat can be recovered in multi-stream exchangers in the quench section for the avoidance of heavy hydrocarbon condensates.

At the same time, to reduce fuel gas consumption and flue gas emissions, it is of equal interest to maximize the air preheating in liquid feed furnaces as in gas feed furnaces. Despite these additional/tightened constraints, embodiments of the present invention are applicable to liquid feed furnaces, in particular by using to an increased extent an indirect cracked gas heat recovery via steam generation by cracked cas cooling and condensation of at least a part of this steam for process gas (process steam and/or hydrocarbon feed) preheating at lower temperatures. This is advantageous since the preheating at temperatures below 300 °C covers most of the additional vaporization enthalpy of the liquid feedstock.

Conservative embodiments of the present invention for liquid feed furnaces may all feature a comparatively high usage of indirect feed preheating using cracked gas heat, compared to the setups for gaseous feed. Although one may suppose that the additional feed preheating duty for feed evaporation limits the achievable air preheating levels for liquid furnaces, the invention proves the opposite, since a major portion of the duty for feed preheating is required to evaporate the liquid hydrocarbon feed, which can be achieved by condensation of saturated very high-pressure steam at temperatures well below 300 °C. Furthermore, the reliable design and operation of an alternative, direct feed-effluent exchanger may be hampered further if the feed stream on the coolant side is multiphasic and additionally contains many fouling-prone components.

**Table 1: Performance data for selected embodiments and prior art (gas feed crackers)**

| | Embodiment 1 (gas feed) | Embodiment 2 (gas feed) | Embodiment 3 (gas feed) | Prior art w/o APH (gas feed) | Prior art with low external APH (gas feed) | Prior art with low internal APH (gas feed) |
|---|---|---|---|---|---|---|
| **Flue gas heat usage** | | | | | | |
| Total heat recovered from flue gas stream downstream of radiant section [MW] | 28.9 | 21.2 | 20.8 | 52.7 | 40.6 | 40.9 |
| Total heat transferred from flue gas to air preheating [MW] | 13.3 | 16.8 | 17.1 | 0 | 0 | 7.3 |
| Total heat transferred from flue gas to feed preheating [MW] | 10.8 | 0 | 0 | 28.6 | 20.3 | 20.3 |
| Total heat transferred from flue gas to export steam generation [MW] | 4.8 | 4.3 | 3.7 | 23.9 | 20.2 | 13.3 |
| Relative flue gas heat usage for air preheating [-] | 46 % | 79 % | 82 % | 0% | 0% | 18 % |
| Relative flue gas heat usage for feed preheating [-] | 37 % | 0% | 0% | 54 % | 50 % | 50 % |
| Relative share of feed preheating by flue gas vs. total feed preheating [-] | 38 % | 0% | 0% | 100 % | 71 % | 71 % |
| Relative flue gas heat usage for export steam generation [-] | 17% | 21 % | 18 % | 45% | 50 % | 32 % |
| Export steam temperature [°C] | 380 | 380 | 380 | 520 | 520 | 520 |
| Resulting air preheat temperature [°C] | 612 | 907 | 940 | 28 | 300 | 280 |
| Resulting fuel usage (LHV) [MW] | 66.2 | 54.4 | 53.3 | 105.8 | 83.8 | 84.2 |

| **Cracked gas heat usage** | | | | | | |
|---|---|---|---|---|---|---|
| Total cracked gas flow from radiant section [t/h] | 66.5 | 66.5 | 66.5 | 66.5 | 66.5 | 66.5 |
| Total heat recovered from cracked gas stream downstream of radiant section [MW] | 37.5 | 37.5 | 37.5 | 37.5 | 37.5 | 37.5 |
| Total heat transferred from cracked gas to feed preheating [MW] | 18.1 | 19.9 * | 29.0 | 0 | 8.4 | 8.4 |
| - thereof in direct feed-effluent exchanger [MW] | 18.1 | 10.8 | 19.9 | 0 | 8.4 | 8.4 |
| - thereof in indirect heat exchange [MW] | 0 | 9.1 | 9.1 | 0 | 0 | 0 |
| Total heat transferred from cracked gas to export steam generation [MW] | 19.5 | 17.6 | 8.5 | 37.5 | 29.1 | 29.1 |
| Relative cracked gas heat usage for feed preheating [-] | 48 % | 53% | 77% | 0% | 22 % | 22 % |

| | | | | | | |
|---|---|---|---|---|---|---|
| * = Embodiment 2 includes an additional electric preheater with 8.9 MW duty (see also Fig. 4) | | | | | | |

**Table 2: Performance data for selected embodiments and prior art (liquid feed crackers)**

| | Embodi- ment 4 (liquid feed) | Embodi- ment 5 (liquid feed) | Embodi- ment 6 (liquid feed) | Prior art w/o APH (liquid feed) | Prior art with low APH (liquid feed) |
|---|---|---|---|---|---|
| **Flue gas heat usage** | | | | | |
| Total heat recovered from flue gas stream downstream of radiant section [MW] | 33.1 | 29.5 | 24.0 | 48.7 | 46.2 |
| Total heat transferred from flue gas to air preheating [MW] | 12.3 | 14.3 | 17.2 | 0 | 3.3 |
| Total heat transferred from flue gas to feed preheating [MW] | 17.4 | 12.9 | 4.8 | 30.9 | 30.9 |
| Total heat transferred from flue gas to export steam generation [MW] | 3.2 | 2.3 | 1.9 | 17.6 | 11.8 |
| Relative flue gas heat usage for air preheating [-] | 37 % | 48 % | 72% | 0% | 7% |
| Relative flue gas heat usage for feed preheating [-] | 53% | 44 % | 20 % | 63 % | 67 % |
| Relative share of feed preheating by flue gas vs. total feed preheating [-] | 56 % | 42 % | 16 % | 100% | 100% |
| Relative flue gas heat usage for export steam generation [-] | 10 % | 8 % | 8% | 36 % | 26 % |
| Export steam temperature [°C] | 380 | 380 | 380 | 520 | 520 |
| Resulting air preheat temperature [°C] | 475 | 590 | 820 | 4 | 100 |
| Resulting fuel usage (LHV) [MW] | 65.9 | 60.5 | 51.3 | 97.2 | 89.2 |

| **Cracked gas heat usage** | | | | | |
|---|---|---|---|---|---|
| Total cracked gas flow from radiant section [t/h] | 75.5 | 75.5 | 75.5 | 75.5 | 75.5 |
| Total heat recovered from cracked gas stream downstream of radiant section [MW] | 29.9 | 29.9 | 29.9 | 29.9 | 29.9 |
| Total heat transferred from cracked gas to feed preheating [MW] | 13.5 | 18.1 | 26.2 | 0 | 0 |
| - thereof in direct feed-effluent exchanger [MW] | 0 | 10.5 | 12.7 | 0 | 0 |
| - thereof in indirect heat exchange [MW] | 13.5 | 7.6 | 13.4 | 0 | 0 |
| Total heat transferred from cracked gas to export steam generation [MW] | 16.5 | 11.9 | 3.7 | 29.9 | 29.9 |
| Relative cracked gas heat usage for feed preheating [-] | 45 % | 60 % | 87 % | 0% | 0% |

Rather than reducing the available duty for air preheating, this comparatively high usage of indirect process gas, hydrocarbon feed and/or process steam preheating using cracked gas heat leads to a reduced net export steam quantity, which additionally reduces the relative heat duty used in the convection section for steam superheating and, thus, actually increases the duty available for the internal air preheating segments.

Indeed, as can be seen in the comparison of Tables 1 and 2, the liquid furnace embodiments (see Table 2) use a maximum of 10% of the recovered flue gas heat for export steam superheating, while gas furnace embodiments (see Table 1) use a maximum of 21%. A difference in feedstock therefore does not lead to a change in achievable air preheat temperature, but only in a variation of exported, non-essential utility steam. The relevance of this minor drawback is further alleviated in the context of highly electrified future plant designs, where steam demand is significantly reduced compared to existing designs. Further benefits may result for the design of furnaces with flexible feed supply.

In conventional arrangements, the initial process steam, hydrocarbon feed and/or process steam preheating is exclusively done in the convection section, while only the preheating at higher temperatures above 350°C is done by using cracked gas heat (see WO 2021/052642 A1). In combination with the said downstream positioning of the air preheating bundle within the convection zone, it follows that applying such a conventional design for gaseous and liquid feed furnaces will result in lower air preheating levels for the liquid feed furnace, since more of the residual flue gas heat is consumed for initial feed preheating.

For the sake of completeness, the convection section in liquid feed furnaces typically feature an additional process steam superheater, while the quench section does not include a tertiary quench exchanger, yielding higher cracked gas temperatures at the considered furnace outlet, typically around 380 °C vs. around 200 °C for gas feed furnaces. This is necessary to avoid condensation of heavy condensates when cooling the cracked gas below said level. Hence, in liquid feed crackers, the cracked gas cooling to lower temperatures is done by direct contact cooling with injections of pyrolysis oil/gasoline and/or quench water (related heat recovery at these lower temperatures not within scope of this invention, see nomenclature introduced above).

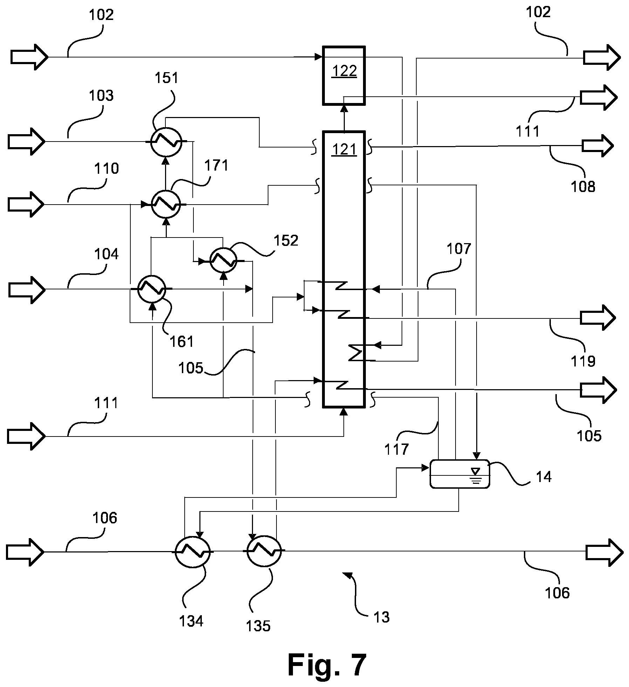

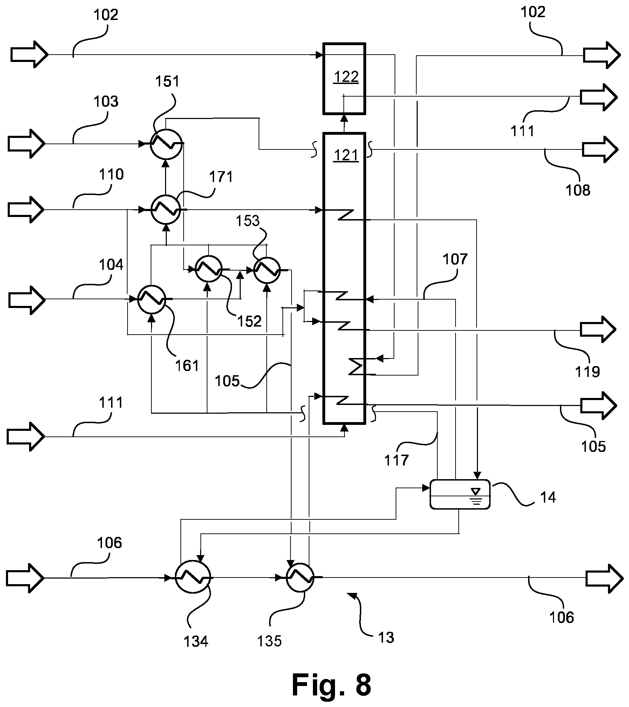

According to the embodiments 1, 2 and 3 shown in Figures 3, 4 and 5, primary, secondary and tertiary quench heat exchangers 131, 132 and 133 are present in the cracked gas heat recovery section 13 through which a cracked gas 106 is passed, while according to embodiment 4 shown in Figure 6, a single primary quench exchanger 134 is used in this connection. According to the embodiments 5 and 6 shown in Figures 7 and 8, a primary quench exchanger 134 in connection with a secondary exchanger 135 is used.

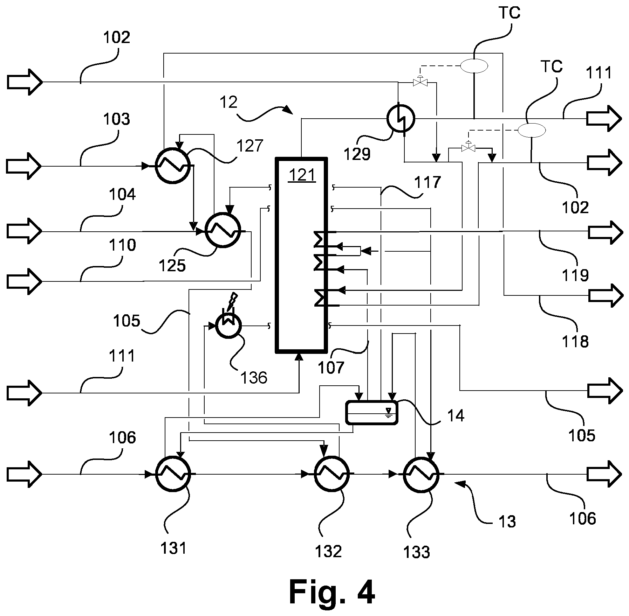

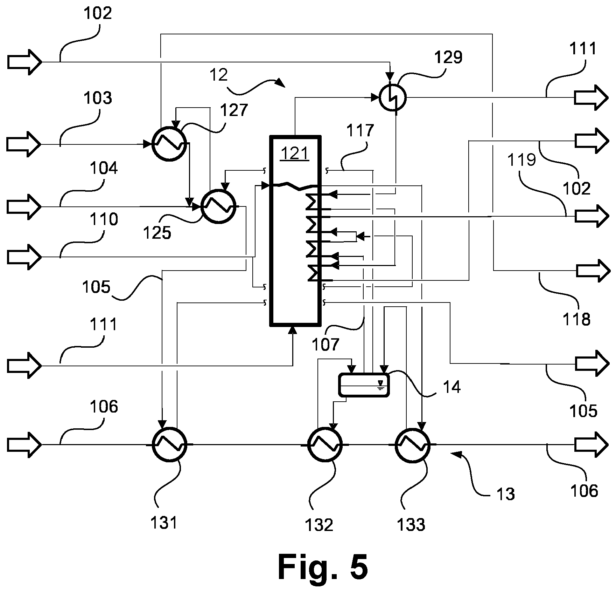

A steam drum is indicated with 14 in all cases. While in the embodiment 1 shown in Figure 3 , a flue gas heat recovery unit is shown as a single unit 12, this may be split, according to the embodiments 2 and 3 shown in Figures 4 and 5 into a (main) flue gas heat recovery sub-unit 121 and an auxiliary flue gas heat exchanger unit 129, and according to the embodiments 4, 5 and 6 shown in Figures 6, 7 and 8, into partial units 121 and 122. A stream of ambient air is used as the oxidizer gas 102 in all embodiments. A hydrocarbon feed 103 is e.g. ethane according to the embodiments 1, 2 and 3 shown in Figures 3, 4 and 5 and may be e.g. naphtha according to the embodiments 4, 5 and 6 shown in Figures 6, 7 and 8. Process steam is indicated with 104 in all cases, and a mixture of feed 103 and process steam, i.e. a "process gas" in the language used herein, is indicated as 105. Further media include boiler feed water 110 and flue gas 111.

Typical air preheating temperatures are 612 °C according to the embodiment 1 shown in Figure 3, 907 °C according to the embodiment 2 shown in Figure 4, 940 °C according to the embodiment 3 shown in Figure 5, 475 °C according to the embodiment 4 shown in Figure 6, 590 °C according to the embodiment 5 shown in Figure 7 and 820 °C according to the embodiment 6 shown in Figure 8.

Turning to the embodiments relating to gaseous hydrocarbon feeds, i.e. the embodiments 1, 2 and 3 shown in Figures 3, 4 and 5, common features and differences of these embodiments will now be described.

According to the embodiment 1 shown in Figure 3, the feed 103 is preheated in the tertiary quench exchanger 133 and thereafter combined with process steam 104. The diluted feed 105, i.e. the process gas, is heated in the secondary quench exchanger 132. According to the embodiments 2 and 3 shown in Figures 4 and 5, in contrast a preheater 127 for feed 103 and a heater 125 for the process gas 105 are present, which are operated with saturated steam 117 from the steam drum 14, forming steam condensate in heater 125 and subcooled steam condensate 118 in preheater 127. In the embodiment 2 shown in Figure 4, the process gas 105 is further heated in the secondary quench exchanger 132 and in an auxiliary (electric) heater 136, while, according to the embodiment 3 shown in Figure 5, the primary quench exchanger 131 is used for this purpose.

The steam drum 14 is operated, in the embodiments 1 and 2 illustrated in Figures 3 and 4, with steam produced in the primary quench exchanger 131 using heat withdrawn from the cracked gas 106 while, in the embodiment 3 shown in Figure 5, the secondary quench exchanger 132 is used for this purpose. In the embodiments shown in Figure 4, boiler feed water 110 is heated in the tertiary quench exchanger 133 before being passed to the steam drum 14. This is also done according to the embodiment 3 shown in Figure 5, but in this embodiment, a preheating of boiler feed water is also performed in the flue gas heat recovery unit. In the embodiments 1, 2 and 3 shown in Figured 3, 4 and 5, steam 107 from the steam drum 14 is also superheated in two steps within the flue gas heat recovery unit 12, with an optional boiler feed water injection in between these two steps, and exported as superheated steam 119.

Preheating of the oxidizer gas 102, e.g. combustion air, is performed in the embodiment 1 shown in Figure 3 in two steps within the flue gas heat recovery unit 12, wherein bypasses around each of the two oxidizer gas heating steps in the flue gas heat recovery unit 12 are illustrated, which may be operated using temperature controllers TC operated on the basis of a flue gas temperature or preheated air temperature.

Preheating of the oxidizer gas 102, i.e. combustion air, is performed in the embodiments 2 and 3 shown in Figures 4 and 5 using an auxiliary flue gas heat exchanger unit 129 that is accounted as part of the flue gas heat recovery section 12 and may be arranged downstream of a (main) flue gas heat recovery sub-unit 121 in the flue gas path, and thereafter in the main flue gas heat recovery sub-unit 121, wherein, in the embodiment shown in Figure 5, two heating steps are performed in the main flue gas heat recovery sub-unit 121 and wherein, in the embodiment 2 shown in Figure 4, bypasses around the auxiliary flue gas heat exchanger 129 and the heating in the main flue gas heat recovery sub-unit 121 are illustrated, which may be operated using temperature controllers TC operated on the basis of a flue gas temperature or preheated air temperature.

Turning to the embodiments 4, 5 and 6 relating to liquid or heavy hydrocarbon feeds, i.e. the embodiments shown in Figures 6, 7 and 8, common features and differences of these embodiments will now be described.

Firstly, in the embodiments 4, 5 and 6 shown in Figures 6, 7 and 8, the steam drum 14 is operated with steam produced in the quench exchanger 134 using heat withdrawn from the cracked gas stream 106. In the embodiments 4, 5 and 6 shown in Figures 6, 7 and 8, furthermore, feed 103 is preheated in a heat exchanger 151 and thereafter further heated in a heat exchanger 152, before being combined with process steam 104. Process steam 104 is, before being combined with feed 103, preheated in a process steam preheater 161.

The process gas 105 is, in the embodiment 4 shown in Figure 6, further heated in a heat exchanger 153 and thereafter in two stages in the heat recovery unit 12 or its sub-unit 121. In contrast, the process gas 105 is, in the embodiment 5 shown in Figure 7, not further heated in a heat exchanger corresponding to heat exchanger 153 as shown in Figure 6, but in the further heat exchanger 135 in the cracked gas heat recovery section 13 and thereafter in one stage in the flue gas heat recovery unit 12 or its sub-unit 121. In the yet further alternative of embodiment 6 shown in Figure 8, process gas 105 is, similarly to the embodiment 4 shown in Figure 6, further heated in a heat exchanger 153, but thereafter, similarly to the embodiment shown in Figure 7, in the further heat exchanger 135 in the cracked gas heat recovery section 13 and one stage in the heat recovery unit 12 or its sub-unit 121.

Boiler feed water 110 is in one part preheated in a boiler feed water preheater 171 and thereafter, in the embodiments 4 and 5 shown in Figures 6 and 7, passed to steam drum 14. In the embodiment 6 shown in Figure 8, this boiler feed water 110 is further heated in the heat recovery unit 12 or its sub-unit 121. A further part of the boiler feed water 110 is injected between two heating stages in the recovery unit 12 or its sub-unit 121 into a steam stream, essentially as illustrated in Figure 1.

Heat exchangers 161, 152, and 153, if present in the case of the latter, are operated in parallel using saturated steam 117 from steam drum 14, and the resulting steam condensate streams are thereafter combined and used in boiler feed water preheater 171 and thereafter in feed preheater 151, where the condensate is subcooled before being exported as subcooled steam condensate stream 108.

According to the embodiments 4, 5 and 6 shown in Figures 6, 7 and 8, combustion air 102 or a stream of oxidizer gas is first preheated in a downstream section of the heat recovery unit 12 or its sub-unit 122, and thereafter in an upstream section of the heat recovery unit 12 or its sub-unit 121.

The combustion air is at least partially preheated inside the flue gas pathway ("internal air preheating"), using an air preheating system composed of a minimum of 2 multistream heat exchangers, with flue gas as hot medium, and air as cold medium. As shown in Figures 3 to 8, in between two air preheating steps, heat is preferably recovered from the flue gas to superheat steam, preferably generated in a steam generating quench exchanger. In some embodiments, in between two air preheating steps, heat may also additionally be recovered from the flue gas to heat other streams, e.g. to preheat feed streams, as shown in Figure 7 for liquid feed cracker setup.

## Claims

1. A method of steam cracking using a steam cracking system comprising one or more fired radiant sections (11),
wherein, during a steam cracking operation, a fuel (101) is combusted with an oxygen containing oxidizer gas (102) to heat the one or more radiant sections (11), forming a flue gas (111),
wherein a process gas (105) is formed from a hydrocarbon feed (103) and process steam (104),
wherein the process gas (105) is passed through one or more cracking coils in the one or more fired radiant sections (11), forming a cracked gas (106),
wherein at least a part of the flue gas (111) is cooled in a flue gas heat recovery section (12),
wherein at least a part of the cracked gas (106) is cooled in a cracked gas heat recovery section (13),
wherein the flue gas heat recovery section (12) comprises one or more heat exchangers,
wherein the cracked gas heat recovery section (13) comprises one or more heat exchangers (131, 132, 133, 134, 135),
wherein the flue gas heat recovery section (12) is configured such as to use, at least during a part of the steam cracking operation, a first proportion of more than 35% of a total amount of heat recovered within the one or more heat exchangers of the flue gas heat recovery section (12) from the flue gas (111) for preheating the oxidizer gas (102),
wherein the cracked gas heat recovery section (13) comprises heat exchange structures which are provided in an amount and a configuration such as to use, at least during the same part of the steam cracking operation, a second proportion of more than 35% of a total amount of heat recovered within the one or more heat exchangers (131, 132, 133, 134) of the cracked gas heat recovery section (13) for preheating the process gas (105) and/or the hydrocarbon feed (103) and/or the process steam (104) used in forming the process gas (105),
wherein the oxidizer gas (102), or a part thereof, is at least partially preheated in a minimum of two separate oxidizer gas preheating stages against the flue gas in the flue gas heat recovery section (12),
wherein the flue gas is used in between the two oxidizer gas preheating stages to superheat high pressure steam at a pressure level between 30 and 175 bar absolute pressure,
wherein saturated steam generated in a quench exchanger is used to preheat the process gas (105) and/or the hydrocarbon feed (103) and/or the process steam (104) used in forming the process gas (105), and
wherein steam condensate collected from said preheating using saturated steam is used for additional preheating of the process gas (105) and/or the hydrocarbon feed (103) and/or the process steam (104) used in forming the process gas (105) at a lower temperature level.

2. The method according to claim 1, wherein the first proportion and the second proportion are, independently from each other, proportions of more than 35%, more than 40%, more than 45%, or more than 50%.

3. The method according to claim 1 or 2, wherein the oxidizer gas (102) is preheated to a temperature exceeding 400 °C, 500 °C, 600 °C, or 700°C.

4. The method according to any one of the preceding claims, wherein steam is superheated in the flue gas heat recovery section (12), wherein said superheating of steam in the flue gas heat recovery section (12) is limited to a maximum temperature of 450 °C, 400 °C or 350°C and/or resulting in a dew point margin of maximally 150 K, 120 K, 80 K, or 60 K, wherein no further steam superheating is performed before exporting the steam from the flue gas heat recovery section (12) or the steam cracking system, and wherein less than 25 %, 23 %, or 21 % of the heat recovered in the flue gas heat recovery section (12) is used for generating high-pressure export steam.

5. The method according to any one of the preceding claims, wherein steam exported from the flue gas heat recovery section (12) or the steam cracking system is passed in a proportion of more than 60%, 70%, or 80% via conduits, pressure-reducing valves and/or nozzles, with or without prior desuperheating, and/or without prior extraction of usable mechanical work, to one or more steam-condensating heat exchangers.

6. The method according to any one of the preceding claims, wherein steam exported from the flue gas heat recovery section (12) or the steam cracking system is not used in steam turbine drives delivering shaft powers of more than 5, 3, or 1 Megawatts.

7. The method according to any one of the preceding claims, wherein the process gas and/or the hydrocarbon feed (103) and/or the process steam (104) used in forming the process gas (105) is heated using recovered cracked gas heat at least partially using a feed-effluent exchanger.

8. The method according to claim 7, wherein the feed-effluent exchanger is located downstream of a steam-generating quench exchanger in a flow path of the cracked gas.

9. The method according to any one of the preceding claims, wherein a final preheating of the process gas (105), or a part thereof, is performed using an electrical superheater, before the mixture enters one or more cracking coils in the one or more fired radiant sections.

10. A steam cracking system comprising:
one or more fired radiant sections (11),
means configured to combust, during a steam cracking operation, a fuel (101) with an oxidizer gas (102) to heat the one or more fired radiant sections (11), forming a flue gas (111),
means configured to form a process gas (105) from a hydrocarbon feed (103) and steam (104), means configured to pass the process gas (105) through one or more cracking coils in the one or more fired radiant sections, forming a cracked gas (106), and
a flue gas heat recovery section (12) configured to cool at least a part of the flue gas (111), a cracked gas heat recovery section (13) configured to cool at least a part of the cracked gas (106),
wherein the flue gas heat recovery section (12) and the cracked gas heat recovery section (13) each comprise one or more heat exchangers (131, 132, 133, 134),
wherein the flue gas heat recovery section (12) is configured such as to use, at least during a part of the steam cracking operation, a first proportion of more than 35% of a total amount of heat recovered within the one or more heat exchangers of the flue gas heat recovery section (12) from the flue gas (111) for preheating the oxidizer gas (102),
wherein the cracked gas heat recovery section (13) is comprises heat exchange structures which are provided in an amount and in a configuration configured such as to use, at least during the same part of the steam cracking operation, a second proportion of more than 35% of a total amount of heat recovered within the one or more heat exchangers (131, 132, 133, 134) of the cracked gas heat recovery (13) section for preheating the process gas (105) and/or the hydrocarbon feed (103) and/or the process steam (104) used in forming the process gas (105),
wherein the steam cracking system comprises a minimum of two separate oxidizer gas preheating stages configured to at least partially preheat the oxidizer gas (102), or a part thereof, against the flue gas in the flue gas heat recovery section (12),
wherein the steam cracking system is configured to use the flue gas in between the two oxidizer gas preheating stages to superheat high pressure steam at a pressure level between 30 and 175 bar absolute pressure,
wherein the steam cracking system is configured to use saturated steam generated in a quench exchanger to preheat the process gas (105) and/or the hydrocarbon feed (103) and/or the process steam (104) used in forming the process gas (105), and
wherein the steam cracking system is configured to use condensate collected from said preheating using saturated steam for additional preheating of the process gas (105) and/or the hydrocarbon feed (103) and/or the process steam (104) used in forming the process gas (105) at a lower temperature level.

11. The system according to claim 10 configured to perform a method according to any one of the preceding claims.

## Patentansprüche

1. Verfahren zum Dampfspalten unter Verwendung eines Dampfspaltsystems, umfassend einen oder mehrere befeuerte Strahlungsabschnitte (11),
wobei während eines Dampfspaltbetriebs ein Brennstoff (101) mit einem sauerstoffhaltigen Oxidationsgas (102) verbrannt wird, um den einen oder die mehreren Strahlungsabschnitte (11) zu erwärmen, wodurch ein Rauchgas (111) gebildet wird,
wobei ein Prozessgas (105) aus einem Kohlenwasserstoffeinsatzstoff (103) und Prozessdampf (104) gebildet wird,
wobei das Prozessgas (105) durch eine oder mehrere Spalt-Coils in dem einen oder den mehreren befeuerten Strahlungsabschnitten (11) geleitet wird, wodurch ein Spaltgas (106) gebildet wird,
wobei mindestens ein Teil des Rauchgases (111) in einem Rauchgaswärmerückgewinnungsabschnitt (12) gekühlt wird,
wobei mindestens ein Teil des Spaltgases (106) in einem Spaltgaswärmerückgewinnungsabschnitt (13) gekühlt wird,
wobei der Rauchgaswärmerückgewinnungsabschnitt (12) einen oder mehrere Wärmetauscher umfasst,
wobei der Spaltgaswärmerückgewinnungsabschnitt (13) einen oder mehrere Wärmetauscher (131, 132, 133, 134, 135) umfasst,
wobei der Rauchgaswärmerückgewinnungsabschnitt (12) konfiguriert ist, um mindestens während eines Teils des Dampfspaltbetriebs einen ersten Anteil von mehr als 35 % einer Gesamtmenge an Wärme, die innerhalb des einen oder der mehreren Wärmetauscher des Rauchgaswärmerückgewinnungsabschnitts (12) aus dem Rauchgas (111) zurückgewonnen wird, zum Vorwärmen des Oxidationsgases (102) zu verwenden,
wobei der Spaltgaswärmerückgewinnungsabschnitt (13) Wärmetauschstrukturen umfasst, die in einer Menge und einer Konfiguration bereitgestellt sind, um mindestens während desselben Teils des Dampfspaltbetriebs einen zweiten Anteil von mehr als 35 % einer Gesamtmenge der innerhalb des einen oder der mehreren Wärmetauscher (131, 132, 133, 134) des Spaltgaswärmerückgewinnungsabschnitts (13) zurückgewonnenen Wärme zum Vorwärmen des Prozessgases (105) und/oder des Kohlenwasserstoffeinsatzstoffs (103) und/oder des Prozessdampfs (104), die zur Bildung des Prozessgases (105) verwendet werden, zu verwenden,
wobei das Oxidationsgas (102) oder ein Teil davon in mindestens zwei separaten Oxidationsgasvorwärmstufen gegen das Rauchgas in dem Rauchgaswärmerückgewinnungsabschnitt (12) mindestens teilweise vorgewärmt wird,
wobei das Rauchgas zwischen den zwei Oxidationsgasvorwärmstufen verwendet wird, um Hochdruckdampf bei einem Druckniveau zwischen 30 und 175 bar Absolutdruck zu überhitzen,
wobei Sattdampf, der in einem Quenchtauscher erzeugt wird, zum Vorwärmen des Prozessgases (105) und/oder des Kohlenwasserstoffeinsatzstoffs (103) und/oder des Prozessdampfes (104), die zur Bildung des Prozessgases (105) verwendet werden, verwendet wird, und
wobei Dampfkondensat, das aus der Vorwärmung unter Verwendung von Sattdampf gesammelt wird, zur zusätzlichen Vorwärmung des Prozessgases (105) und/oder des Kohlenwasserstoffeinsatzstoffs (103) und/oder des Prozessdampfes (104), die zur Bildung des Prozessgases (105) verwendet werden, bei einem niedrigeren Temperaturniveau verwendet wird.

2. Verfahren nach Anspruch 1, wobei der erste Anteil und der zweite Anteil unabhängig voneinander Anteile von mehr als 35 %, mehr als 40 %, mehr als 45 % oder mehr als 50 % sind.

3. Verfahren nach Anspruch 1 oder 2, wobei das Oxidationsgas (102) auf eine Temperatur von mehr als 400 °C, 500 °C, 600 °C oder 700 °C vorgewärmt wird.

4. Verfahren nach einem der vorstehenden Ansprüche, wobei Dampf in dem Rauchgaswärmerückgewinnungsabschnitt (12) überhitzt wird, wobei die Überhitzung von Dampf in dem Rauchgaswärmerückgewinnungsabschnitt (12) auf eine Maximaltemperatur von 450 °C, 400 °C oder 350 °C begrenzt ist und/oder zu einem Taupunktabstand von maximal 150 K, 120 K, 80 K oder 60 K führt, wobei keine weitere Dampfüberhitzung vor dem Export des Dampfes aus dem Rauchgaswärmerückgewinnungsabschnitt (12) oder dem Dampfspaltsystem durchgeführt wird, und wobei weniger als 25 %, 23 % oder 21 % der in dem Rauchgaswärmerückgewinnungsabschnitt (12) zurückgewonnenen Wärme zur Erzeugung von Hochdruckexportdampf verwendet wird.

5. Verfahren nach einem der vorstehenden Ansprüche, wobei aus dem Rauchgaswärmerückgewinnungsabschnitt (12) oder dem Dampfspaltsystem exportierter Dampf zu einem Anteil von mehr als 60 %, 70 % oder 80 % über Leitungen, Druckminderventile und/oder Düsen, mit oder ohne vorherige Entüberhitzung und/oder ohne vorherige Gewinnung nutzbarer mechanischer Arbeit, zu einem oder mehreren dampfkondensierenden Wärmetauschern geleitet wird.

6. Verfahren nach einem der vorstehenden Ansprüche, wobei aus dem Rauchgaswärmerückgewinnungsabschnitt (12) oder dem Dampfspaltsystem exportierter Dampf nicht in Dampfturbinenantrieben verwendet wird, die Wellenleistungen von mehr als
5, 3 oder 1 Megawatt aufweisen.

7. Verfahren nach einem der vorstehenden Ansprüche, wobei das Prozessgas und/oder der Kohlenwasserstoffeinsatzstoff (103) und/oder der Prozessdampf (104), die zur Bildung des Prozessgases (105) verwendet werden, unter Verwendung von zurückgewonnener Spaltgaswärme mindestens teilweise unter Verwendung eines Zulauf-Ablauf-Tauschers erwärmt werden.

8. Verfahren nach Anspruch 7, wobei der Zulauf-Ablauf-Tauscher stromabwärts eines dampferzeugenden Quenchtauschers in einem Strömungsweg des Spaltgases angeordnet ist.

9. Verfahren nach einem der vorstehenden Ansprüche, wobei eine abschließende Vorwärmung des Prozessgases (105) oder eines Teils davon unter Verwendung eines elektrischen Überhitzers durchgeführt wird, bevor das Gemisch in eine oder mehrere Spalt-Coils in dem einen oder den mehreren befeuerten Strahlungsabschnitten eintritt.

10. Dampfspaltsystem, umfassend:
einen oder mehrere befeuerte Strahlungsabschnitte (11),
Mittel, die konfiguriert sind, um während eines Dampfspaltbetriebs einen Brennstoff (101) mit einem Oxidationsgas (102) zu verbrennen, um den einen oder die mehreren befeuerten Strahlungsabschnitte (11) zu erhitzen, wobei ein Rauchgas (111) gebildet wird,
Mittel, die konfiguriert sind, um ein Prozessgas (105) aus einem Kohlenwasserstoffeinsatzstoff (103) und Dampf (104) zu bilden, Mittel, die konfiguriert sind, um das Prozessgas (105) durch eine oder mehrere Spalt-Coils in dem einen oder den mehreren befeuerten Strahlungsabschnitten zu leiten, wodurch ein Spaltgas (106) gebildet wird, und
einen Rauchgaswärmerückgewinnungsabschnitt (12), der konfiguriert ist, um mindestens einen Teil des Rauchgases (111) zu kühlen, einen Spaltgaswärmerückgewinnungsabschnitt (13), der konfiguriert ist, um mindestens einen Teil des Spaltgases (106) zu kühlen,
wobei der Rauchgaswärmerückgewinnungsabschnitt (12) und der Spaltgaswärmerückgewinnungsabschnitt (13) jeweils einen oder mehrere Wärmetauscher (131, 132, 133, 134) umfassen,
wobei der Rauchgaswärmerückgewinnungsabschnitt (12) konfiguriert ist, um mindestens während eines Teils des Dampfspaltbetriebs einen ersten Anteil von mehr als 35 % einer Gesamtmenge an Wärme, die innerhalb des einen oder der mehreren Wärmetauscher des Rauchgaswärmerückgewinnungsabschnitts (12) aus dem Rauchgas (111) zurückgewonnen wird, zum Vorwärmen des Oxidationsgases (102) zu verwenden,
wobei der Spaltgaswärmerückgewinnungsabschnitt (13) Wärmetauschstrukturen umfasst, die in einer Menge und einer Konfiguration bereitgestellt sind, die konfiguriert sind, um mindestens während desselben Teils des Dampfspaltbetriebs einen zweiten Anteil von mehr als 35 % einer Gesamtmenge der innerhalb des einen oder der mehreren Wärmetauscher (131, 132, 133, 134) des Spaltgaswärmerückgewinnungsabschnitts (13) zurückgewonnenen Wärme zum Vorwärmen des Prozessgases (105) und/oder des Kohlenwasserstoffeinsatzstoffs (103) und/oder des Prozessdampfs (104), die zur Bildung des Prozessgases (105) verwendet werden, zu verwenden,
wobei das Dampfspaltsystem mindestens zwei separate Oxidationsgasvorwärmstufen umfasst, die konfiguriert sind, um das Oxidationsgas (102) oder einen Teil davon gegen das Rauchgas in dem Rauchgaswärmerückgewinnungsabschnitt (12) mindestens teilweise vorzuwärmen,
wobei das Dampfspaltsystem konfiguriert ist, um das Rauchgas zwischen den zwei Oxidationsgasvorwärmstufen zu verwenden, um Hochdruckdampf bei einem Druckniveau zwischen 30 und 175 bar Absolutdruck zu überhitzen,
wobei das Dampfspaltsystem konfiguriert ist, um Sattdampf, der in einem Quenchtauscher erzeugt wird, zu verwenden, um das Prozessgas (105) und/oder den Kohlenwasserstoffeinsatzstoff (103) und/oder den Prozessdampf (104), die zur Bildung des Prozessgases (105) verwendet werden, vorzuwärmen, und
wobei das Dampfspaltsystem konfiguriert ist, um aus der Vorwärmung unter Verwendung von Sattdampf gesammeltes Kondensat zur zusätzlichen Vorwärmung des Prozessgases (105) und/oder des Kohlenwasserstoffeinsatzstoffs (103) und/oder des Prozessdampfes (104), die zur Bildung des Prozessgases (105) verwendet werden, bei einem niedrigeren Temperaturniveau zu verwenden.

11. System nach Anspruch 10, das konfiguriert ist, um ein Verfahren nach einem der vorstehenden Ansprüche durchzuführen.

## Revendications

1. Procédé de vapocraquage à l'aide d'un système de vapocraquage comprenant une ou plusieurs sections rayonnantes à combustion (11),
dans lequel, au cours d'une opération de vapocraquage, un combustible (101) est brûlé avec un gaz oxydant contenant de l'oxygène (102) pour chauffer la ou les sections rayonnantes (11), formant un gaz de combustion (111),
dans lequel un gaz de procédé (105) est formé à partir d'une charge d'hydrocarbures (103) et de vapeur de procédé (104),
dans lequel le gaz de procédé (105) est amené à passer à travers un ou plusieurs serpentins de craquage dans la ou les sections rayonnantes à combustion (11), formant un gaz craqué (106),
dans lequel au moins une partie du gaz de combustion (111) est refroidie dans une section de récupération de chaleur de gaz de combustion (12),
dans lequel au moins une partie du gaz craqué (106) est refroidie dans une section de récupération de chaleur de gaz craqué (13),
dans lequel la section de récupération de chaleur de gaz de combustion (12) comprend un ou plusieurs échangeurs de chaleur,
dans lequel la section de récupération de chaleur de gaz craqué (13) comprend un ou plusieurs échangeurs de chaleur (131, 132, 133, 134, 135),
dans lequel la section de récupération de chaleur de gaz de combustion (12) est conçue de manière à utiliser, au moins au cours d'une partie de l'opération de vapocraquage, une première proportion de plus de 35 % d'une quantité totale de chaleur récupérée au sein du ou des échangeurs de chaleur de la section de récupération de chaleur de gaz de combustion (12) à partir du gaz de combustion (111) pour préchauffer le gaz oxydant (102),
dans lequel la section de récupération de chaleur de gaz craqué (13) comprend des structures d'échange de chaleur qui sont prévues en une quantité et dans une configuration telles qu'elles permettent d'utiliser, au moins au cours de la même partie de l'opération de vapocraquage, une seconde proportion de plus de 35 % d'une quantité totale de chaleur récupérée au sein du ou des échangeurs de chaleur (131, 132, 133, 134) de la section de récupération de chaleur de gaz craqué (13) pour préchauffer le gaz de procédé (105) et/ou la charge d'hydrocarbures (103) et/ou la vapeur de procédé (104) utilisée pour former le gaz de procédé (105),
dans lequel le gaz oxydant (102), ou une partie de celui-ci, est au moins partiellement préchauffé dans au minimum deux étages de préchauffage de gaz oxydant distincts contre le gaz de combustion dans la section de récupération de chaleur de gaz de combustion (12),
dans lequel le gaz de combustion est utilisé entre les deux étages de préchauffage de gaz oxydant pour surchauffer de la vapeur à haute pression à un niveau de pression compris entre 30 et 175 bar de pression absolue,
dans lequel la vapeur saturée générée dans un échangeur de trempe est utilisée pour préchauffer le gaz de procédé (105) et/ou la charge d'hydrocarbures (103) et/ou la vapeur de procédé (104) utilisée pour former le gaz de procédé (105), et
dans lequel le condensat de vapeur collecté à partir dudit préchauffage à l'aide de vapeur saturée est utilisé pour un préchauffage supplémentaire du gaz de procédé (105) et/ou de la charge d'hydrocarbures (103) et/ou de la vapeur de procédé (104) utilisée pour former le gaz de procédé (105) à un niveau de température plus bas.

2. Procédé selon la revendication 1, dans lequel la première proportion et la seconde proportion sont, indépendamment l'une de l'autre, des proportions de plus de 35 %, de plus de 40 %, de plus de 45 %, ou de plus de 50 %.

3. Procédé selon la revendication 1 ou 2, dans lequel le gaz oxydant (102) est préchauffé à une température dépassant 400 °C, 500 °C, 600 °C, ou 700 °C.

4. Procédé selon l'une quelconque des revendications précédentes, dans lequel de la vapeur est surchauffée dans la section de récupération de chaleur de gaz de combustion (12), dans lequel ladite surchauffe de la vapeur dans la section de récupération de chaleur de gaz de combustion (12) est limitée à une température maximale de 450 °C, 400 °C ou 350 °C et/ou résultant en une marge de point de rosée au maximum de 150 K, 120 K, 80 K, ou 60 K, dans lequel aucune surchauffe supplémentaire de la vapeur n'est effectuée avant l'exportation de la vapeur depuis la section de récupération de chaleur de gaz de combustion (12) ou le système de vapocraquage, et dans lequel moins de 25 %, 23 %, ou 21 % de la chaleur récupérée dans la section de récupération de chaleur de gaz de combustion (12) est utilisée pour générer de la vapeur d'exportation à haute pression.

5. Procédé selon l'une quelconque des revendications précédentes, dans lequel la vapeur exportée depuis la section de récupération de chaleur de gaz de combustion (12) ou le système de vapocraquage est acheminée dans une proportion de plus de 60 %, 70 %, ou 80 % par le biais de conduits, réducteurs de pression et/ou buses, avec ou sans désurchauffe préalable, et/ou sans extraction préalable de travail mécanique utilisable, vers un ou plusieurs échangeurs de chaleur de condensation de vapeur.

6. Procédé selon l'une quelconque des revendications précédentes, dans lequel la vapeur exportée depuis la section de récupération de chaleur de gaz de combustion (12) ou le système de vapocraquage n'est pas utilisée dans des entraînements de turbine à vapeur délivrant des puissances d'arbre supérieures à
5, 3, ou 1 mégawatts.

7. Procédé selon l'une quelconque des revendications précédentes, dans lequel le gaz de procédé et/ou la charge d'hydrocarbures (103) et/ou la vapeur de procédé (104) utilisée pour former le gaz de procédé (105) sont chauffés à l'aide de chaleur de gaz craqué récupérée, au moins partiellement à l'aide d'un échangeur charge-effluent.

8. Procédé selon la revendication 7, dans lequel l'échangeur charge-effluent est situé en aval d'un échangeur de trempe générant de la vapeur dans un chemin d'écoulement du gaz craqué.

9. Procédé selon l'une quelconque des revendications précédentes, dans lequel un préchauffage final du gaz de procédé (105), ou d'une partie de celui-ci, est effectué à l'aide d'un surchauffeur électrique, avant que le mélange n'entre dans un ou plusieurs serpentins de craquage dans la ou les sections rayonnantes à combustion.

10. Système de vapocraquage comprenant :
une ou plusieurs sections rayonnantes à combustion (11),
un moyen conçu pour brûler, au cours d'une opération de vapocraquage, un combustible (101) avec un gaz oxydant (102) pour chauffer la ou les sections rayonnantes à combustion (11), formant un gaz de combustion (111),
un moyen conçu pour former un gaz de procédé (105) à partir d'une charge d'hydrocarbures (103) et de vapeur (104), un moyen conçu pour faire passer le gaz de procédé (105) à travers un ou plusieurs serpentins de craquage dans la ou les sections rayonnantes à combustion, formant un gaz craqué (106), et
une section de récupération de chaleur de gaz de combustion (12) conçue pour refroidir au moins une partie du gaz de combustion (111), une section de récupération de chaleur de gaz craqué (13) conçue pour refroidir au moins une partie du gaz craqué (106),
dans lequel la section de récupération de chaleur de gaz de combustion (12) et la section de récupération de chaleur de gaz craqué (13) comprennent chacune un ou plusieurs échangeurs de chaleur (131, 132, 133, 134),
dans lequel la section de récupération de chaleur de gaz de combustion (12) est conçue de manière à utiliser, au moins au cours d'une partie de l'opération de vapocraquage, une première proportion de plus de 35 % d'une quantité totale de chaleur récupérée au sein du ou des échangeurs de chaleur de la section de récupération de chaleur de gaz de combustion (12) à partir du gaz de combustion (111) pour préchauffer le gaz oxydant (102),
dans lequel la section de récupération de chaleur de gaz craqué (13) comprend des structures d'échange de chaleur qui sont prévues en une quantité et dans une configuration configurées telles qu'elles permettent d'utiliser, au moins au cours de la même partie de l'opération de vapocraquage, une seconde proportion de plus de 35 % d'une quantité totale de chaleur récupérée au sein du ou des échangeurs de chaleur (131, 132, 133, 134) de la section de récupération de chaleur de gaz craqué (13) pour préchauffer le gaz de procédé (105) et/ou la charge d'hydrocarbures (103) et/ou la vapeur de procédé (104) utilisée pour former le gaz de procédé (105),
dans lequel le système de vapocraquage comprend au minimum deux phases distinctes de préchauffage de gaz oxydant conçues pour préchauffer au moins partiellement le gaz oxydant (102), ou une partie de celui-ci, contre le gaz de combustion dans la section de récupération de chaleur de gaz de combustion (12),
dans lequel le système de vapocraquage est conçu pour utiliser le gaz de combustion entre les deux étages de préchauffage de gaz oxydant pour surchauffer de la vapeur à haute pression à un niveau de pression compris entre 30 et 175 bar de pression absolue,
dans lequel le système de vapocraquage est conçu pour utiliser de la vapeur saturée générée dans un échangeur de trempe pour préchauffer le gaz de procédé (105) et/ou la charge d'hydrocarbures (103) et/ou la vapeur de procédé (104) utilisée pour former le gaz de procédé (105), et
dans lequel le système de vapocraquage est conçu pour utiliser le condensat collecté à partir dudit préchauffage à l'aide de vapeur saturée pour un préchauffage supplémentaire du gaz de procédé (105) et/et/ou de la charge d'hydrocarbures (103) et/ou de la vapeur de procédé (104) utilisée pour former le gaz de procédé (105) à un niveau de température plus bas.

11. Système selon la revendication 10, configuré pour effectuer un procédé selon l'une quelconque des revendications précédentes.
